# EUROPEAN PATENT APPLICATION

(11) **EP 4 722 364 A1**
(43) Date of publication of application: **08.04.2026**
(21) Application number: 24814341.4
(22) Date of filing: 24.05.2024
(51) Int. Cl.: C12N 15/113, A61K 31/713, A61P 35/00, A61P 25/00, A61P 37/00, A61P 7/00, A61P 9/00, A61P 13/12, A61P 27/02

(54) **DSRNA MOLECULE FOR INHIBITING C5 GENE EXPRESSION, AND USE THEREOF**

(30) Priority: 26.05.2023 CN 202310603955
(71) Applicant: CSPC Zhongqi Pharmaceutical Technology (Shijiazhuang) Co., Ltd., Shijiazhuang, Hebei 050035 (CN)
(72) Inventor: ZHANG, Xueyan, Shijiazhuang, Hebei 050035 (CN); WANG, Lingyu, Shijiazhuang, Hebei 050035 (CN); SU, Xiaoye, Shijiazhuang, Hebei 050035 (CN); SHI, Yanjun, Shijiazhuang, Hebei 050035 (CN); SHAO, Yu, Shijiazhuang, Hebei 050035 (CN); CHEN, Huiyu, Shijiazhuang, Hebei 050035 (CN)
(74) Representative: Studio Torta S.p.A.
(86) International application number: PCT/CN2024/095250
(87) International publication number: WO 2024/245144

(57) **Abstract**

Provided are a modified dsRNA molecule and a use thereof. Specifically provided is a dsRNA molecule for inhibiting C5 gene expression, comprising a sense strand and an antisense strand which are complementary to each other to form a double-stranded region, wherein the sense strand and/or the antisense strand comprise(s) 15 to 25 nucleotides or consist(s) of 15 to 25 nucleotides. The dsRNA molecule can be used for treating and/or preventing C5 gene-mediated diseases.

## Description

### CROSS-REFERENCE TO RELATED APPLICATIONS

The present invention claims priority to Chinese application No. 202310603955.5, titled "DSRNA MOLECULE FOR INHIBITING C5 GENE EXPRESSION, AND USE THEREOF" and filed with the China National Intellectual Property Administration on May 26, 2023, the content of which is incorporated herein by reference in its entirety.

### TECHNICAL FIELD

The present invention pertains to the field of molecular biology, and relates to a modified dsRNA molecule and use thereof, specifically to a dsRNA molecule for inhibiting complement component 5 (C5) gene expression and a pharmaceutical composition comprising the same, as well as to a method for reducing the gene expression level of complement C5 using the dsRNA molecule or the pharmaceutical composition.

### BACKGROUND

RNA interference (RNAi) is a highly conserved phenomenon during evolution in which double-stranded RNAs (dsRNAs) induce efficient and specific degradation of homologous mRNAs. RNAi is a ubiquitous surveillance mechanism in eukaryotic organisms that functions to resist viral invasion, suppress transposon activity, and regulate gene expression. Small interfering RNAs (siRNAs), a class of short double-stranded RNA molecules with a length of 19 to 30 bp, are important tools in RNAi technology. Within organisms, after entering cells, a dsRNA is specifically recognized by a Dicer enzyme and cleaved into small RNA fragments (i.e., dsRNA) of 21 to 23 nucleotides in length. These resulting dsRNA fragments unwind into single strands that assemble with certain proteins to form complexes (referred to as RISC). The RISC can bind to an mRNA that is complementary to the dsRNA within cells and cleave the mRNA, causing the mRNA to degrade. This prevents protein synthesis, leading to the phenomenon called gene "silencing". In industrial production, dsRNA is typically chemically synthesized and subsequently modified to further improve the stability and effectiveness of dsRNA drugs.

Complements are a group of heat-labile proteins that gain enzymatic activity after activation and are widely present in sera and tissue fluids and on cell membrane surfaces, including more than 30 soluble proteins and membrane-bound proteins, collectively referred to as the complement system. Under physiological conditions, most complement components exist as inactive enzyme precursors that undergo activation through a series of cascade enzymatic reactions triggered by different activators (bacteria, antigen-antibody complexes, etc.), exhibiting various biological activities and participating in specific and non-specific immune mechanisms of the organism, manifested as anti-microbial defense responses, immunoregulation, and immune pathologymediated damage responses. Excessive activation or undue inhibition of the complement system plays an important and extensive role in the pathogenesis of a wide range of diseases, including acute inflammatory diseases such as ophthalmic diseases and periodontal diseases, and chronic diseases such as cancer, autoimmune diseases, neurodegenerative diseases, kidney diseases and chronic hemolytic diseases. There are three known complement activation pathways, i.e., the classical pathway, the alternative pathway, and the lectin pathway. Complement protein C5 is at the terminal of the complement cascade reaction, and all pathways of complement activation lead to the cleavage of C5 molecules, thereby producing anaphylatoxin C5a and C5b. C5a binds to its receptor to exert primary pro-inflammatory activity, and C5b binds to four other complement proteins (C6, C7, C8 and C9) to form the complex C5b-9 that causes cell lysis by forming the membrane attack complex (MAC). Sublytic MAC and soluble C5b-9 also have numerous nonlytic immune functions. C5a and C5b-9, the two complement effectors generated from C5 cleavage, are key components of the complement system responsible for propagating and/or initiating pathology in various diseases, such as ophthalmic diseases, including age-related macular degeneration (AMD); central/peripheral nervous system (CNS/PNS) diseases, including Alzheimer's disease (AD), and myasthenia gravis (gMG); renal diseases, including atypical hemolytic uremic syndrome (aHUS), C3 glomerulopathy (C3G) and IgA nephropathy; hematologic diseases, including paroxysmal nocturnal hemoglobinuria (PNH), and thrombotic microangiopathy (TMAs). Therefore, targeting C5 protein can modulate complement signaling activated by the three different complement pathways.

There remains an unmet need in the art for alternative or combination therapies for patients suffering from diseases associated with complement component C5.

### SUMMARY OF THE INVENTION

The present invention provides a dsRNA molecule, an agent, a kit and a pharmaceutical composition for inhibiting the gene expression of complement C5, as well as a method and use of the dsRNA molecule, the agent, the kit or the pharmaceutical composition for inhibiting or reducing the gene expression of C5, or preventing or treating complement-mediated diseases or conditions. The dsRNA molecule facilitates the sequence-specific degradation of C5 mRNA by the RNAi mechanism, thereby inhibiting the gene expression of C5 or reducing the gene expression level of C5.

In one aspect, the present invention provides a double-stranded ribonucleic acid (dsRNA) agent for inhibiting the gene expression of complement component C5, comprising a sense strand and an antisense strand, in which the sense strand and/or the antisense strand comprise(s) at least 15 contiguous nucleotides having no more than 3 nucleotide variations relative to any one of the nucleotide sequences of the sense strand and antisense strand as set forth in Tables 2, 6, or 9.

In some embodiments, the present invention provides a dsRNA molecule, comprising a sense strand with the nucleotide sequence as set forth in Table 2, and an antisense strand with the nucleotide sequence as set forth in Table 2. The sense strand and/or the antisense strand comprise(s) or consist(s) of 15 to 25 nucleotides, the antisense strand is complementary to 15, 16, 17, 18, 19, 20, 21, 22, 23, 24, or 25 contiguous nucleotides of the sequence in the sequence table, the length of the double-stranded region is 15 to 25 bp, preferably 19 to 21 bp, and at least one nucleotide in the dsRNA molecule comprises a modification.

In some embodiments, the sense strand and the antisense strand comprise sequences of a dsRNA molecule selected from the group consisting of 5-E04, 5-E05, 7-E04, 7-E05, 17- E04, 17-E05, 32-E04, 32-E05, 85-E04, 85-E05, GAL-E5, GAL-E17, GAL-E85-1, and GAL-E85.

In some embodiments, the modification is one or more selected from the group consisting of a a locked nucleic acid (LNA) modification, an open or unlocked nucleic acid (UNA) modification, a 2'-methoxyethyl modification, a 2'-O-methyl modification, a 2'-O-allyl modification, a 2'-C-allyl modification, a 2'-fluoro modification, a 2'-deoxy modification, a 2'-hydroxyl modification, a phosphorothioate backbone modification, a DNA modification, a fluorescent probe modification, and a ligand modification.

In some embodiments, the naked sequence of the sense strand of the dsRNA molecule is as set forth in (A1), (A2) or (A3) as follows, and the naked sequence of the antisense strand of the dsRNA molecule is as set forth in (A4), (A5) or (A6) as follows:
(A1): GUGAUUCAAGUUUAUGGAUAC (SEQ ID NO: 5),
(A2): UGUGUAUUUGGAAGUUGUAUC (SEQ ID NO: 17),
(A3): GUGAAGAAAUGUUGUUACGAU (SEQ ID NO: 85),
(A4): GUAUCCAUAAACUUGAAUCACAA (SEQ ID NO: 105),
(A5): GAUACAACUUCCAAAUACACAUA (SEQ ID NO: 117), and
(A6): AUCGUAACAACAUUUCUUCACUA (SEQ ID NO: 185).

In some embodiments, the dsRNA molecule comprises modifications as described below for the sense strand and the antisense strand:
(i) the sense strand has a length of 17 to 21 nt, such as 21 nt, the sense strand consists of alternating 2'-O-methyl modified regions and 2'-fluoro modified regions, wherein each modified region is 1 to 3 nucleotides in length, and the sense strand has identical modifications for the first modified region from the 5' end and the first modified region from the 3' end; and
(ii) the antisense strand has a length of 19 to 23 nt, such as 23 nt, the antisense strand consists of alternating 2'-O-methyl modified regions, 2'-fluoro modified regions, unmodified regions or DNA regions, wherein each modified region is 1 to 5 nucleotides in length, and the antisense strand has phosphorothioate backbone linkages among the contiguous nucleotides at positions 2 to 5 from the 5' end and among the contiguous nucleotides at positions 1 to 3 from the 3' end.

In some embodiments, the dsRNA molecule comprises modifications as described below for the sense strand and the antisense strand:
(i) the sense strand has a length of 21 nt, has 2'-fluoro modifications at positions 7 and 9 to 11 from the 5' end and 2'-O-methyl modifications at the remaining positions, and has phosphorothioate backbone linkages among the contiguous nucleotides at positions 1 to 3 from the 5' end; and
(ii) the antisense strand has a length of 23 nt, has 2'-fluoro modifications at positions 2, 14, and 16 and optional position 6 from the 5' end and 2'-O-methyl modifications at the remaining positions, and has phosphorothioate backbone linkages among the contiguous nucleotides at positions 1 to 3 from the 5' end and among the contiguous nucleotides at positions 1 to 3 from the 3' end.

In some embodiments, the sense strand of the dsRNA molecule comprises the modifications as defined in Modification Motif i, and the antisense strand of the dsRNA molecule comprises the modifications as defined in Modification Motif iii or Modification Motif iv:
Modification Motif i:
   XmsXmsXmXmXmXmXfXmXfXfXfXmXmXmXmXmXmXmXmXmXm;
Modification Motif iii:
   XmsXfsXmXmXmXfXmXmXmXmXmXmXmXfXmXfXmXmXmXmXmsXmsXm; and
Modification Motif iv:
   XmsXfsXmXmXmXmXmXmXmXmXmXmXmXfXmXfXmXmXmXmXmsXmsXm;
   wherein, for each Modification Motif, individual "X's" represent ribonucleotides from the 5' end to the 3' end of the sense strand in sequence, "Xm" represents a ribonucleotide comprising a 2'-O-methyl modification, "Xf" represents a ribonucleotide comprising a 2'-fluoro modification, and "s" represents a phosphorothioate backbone linkage between two flanking ribonucleotides.

In some embodiments, the sense strand of the dsRNA molecule has a structure as set forth in (B1), (B2), or (B3), and the antisense strand of the dsRNA molecule has a structure as set forth in (B4), (B5), (B6), (B7), (B8), or (B9).
(B1), (B2) or (B3) are (A1), (A2) or (A3) comprising the modifications as defined in Modification Motif i:
(B1): SEQ ID NO: 5 comprising the modifications as defined in Modification Motif i,
(B2): SEQ ID NO: 17comprising the modifications as defined in Modification Motif i, and
(B3): SEQ ID NO: 85 comprising the modifications as defined in Modification Motif i.
(B4), (B5), or (B6) are (A4), (A5) or (A6) comprising the modifications as defined in Modification Motif iii, and (B7), (B8), or (B9) are (A4), (A5) or (A6) comprising the modifications as defined in Modification Motif iv:
(B4): SEQ ID NO: 105 comprising the modifications as defined in Modification Motif iii,
(B5): SEQ ID NO: 117 comprising the modifications as defined in Modification Motif iii,
(B6): SEQ ID NO: 185 comprising the modifications as defined in Modification Motif iii,
(B7): SEQ ID NO: 105 comprising the modifications as defined in Modification Motif iv,
(B8): SEQ ID NO: 117comprising the modifications as defined in Modification Motif iv, and
(B9): SEQ ID NO: 185 comprising the modifications as defined in Modification Motif iv.
For (B1) to (B9):
   Modification Motif i:
      XmsXmsXmXmXmXmXfXmXfXfXfXmXmXmXmXmXmXmXmXmXm;
   Modification Motif iii:
      XmsXfsXmXmXmXfXmXmXmXmXmXmXmXfXmXfXmXmXmXmXmsXmsXm; and
   Modification Motif iv:
      XmsXfsXmXmXmXmXmXmXmXmXmXmXmXfXmXfXmXmXmXmXmsXmsXm;
wherein, for each Modification Motif, individual "X's" represent ribonucleotides from the 5' end to the 3' end of the sense strand in sequence, "Xm" represents a ribonucleotide comprising a 2'-O-methyl modification, "Xf" represents a ribonucleotide comprising a 2'-fluoro modification, and "s" represents a phosphorothioate backbone linkage between two flanking ribonucleotides.

In some preferred embodiments, the dsRNA molecule is selected from the group consisting of:
5-E04, comprising a sense strand of SEQ ID NO: 5 comprising the modifications as defined in Modification Motif i, and an antisense strand of SEQ ID NO: 105 comprising the modifications as defined in Modification Motif iii;
5-E05, comprising a sense strand of SEQ ID NO: 5 comprising the modifications as defined in Modification Motif i, and an antisense strand of SEQ ID NO: 105 comprising the modifications as defined in Modification Motif iv;
7-E04, comprising a sense strand of SEQ ID NO: 7 comprising the modifications as defined in Modification Motif i, and an antisense strand of SEQ ID NO: 107 comprising the modifications as defined in Modification Motif iii;
7-E05, comprising a sense strand of SEQ ID NO: 7 comprising the modifications as defined in Modification Motif i, and an antisense strand of SEQ ID NO: 107 comprising the modifications as defined in Modification Motif iv;
17-E04, comprising a sense strand of SEQ ID NO: 17 comprising the modifications as defined in Modification Motif i, and an antisense strand of SEQ ID NO: 117 comprising the modifications as defined in Modification Motif iii;
17-E05, comprising a sense strand of SEQ ID NO: 17 comprising the modifications as defined in Modification Motif i, and an antisense strand of SEQ ID NO: 117 comprising the modifications as defined in Modification Motif iv;
32-E04, comprising a sense strand of SEQ ID NO: 32 comprising the modifications as defined in Modification Motif i, and an antisense strand of SEQ ID NO: 132 comprising the modifications as defined in Modification Motif iii;
32-E05, comprising a sense strand of SEQ ID NO: 32 comprising the modifications as defined in Modification Motif i, and an antisense strand of SEQ ID NO: 132 comprising the modifications as defined in Modification Motif iv;
85-E04, comprising a sense strand of SEQ ID NO: 85 comprising the modifications as defined in Modification Motif i, and an antisense strand of SEQ ID NO: 185 comprising the modifications as defined in Modification Motif iii; and
85-E05, comprising a sense strand of SEQ ID NO: 85 comprising the modifications as defined in Modification Motif i, and an antisense strand of SEQ ID NO: 185 comprising the modifications as defined in Modification Motif iv.

In some preferred embodiments, the dsRNA molecule is 85-E04, 85-E05, 5-E05, or 17-E05.

In some embodiments, the dsRNA molecule has a ligand modification. The ligand is a component taken up by host cells, and ligand modification can improve the properties of the dsRNA molecule, such as cellular uptake, intracellular targeting, half-life, drug metabolism or pharmacokinetics. In some embodiments, as compared with a dsRNA without the ligand modification, the dsRNA with the ligand modification is directed to a selected target, such as a particular type of tissues, cells or organelles, for example, hepatocytes, and has enhanced affinity or cellular uptake. A ligand modification does not interfere with the activity of the dsRNA.

In some embodiments, the ligand modification is one or more ligand modifications added to the 3' end, the 5' end and/or the interior sequence of the dsRNA molecule.

In some preferred embodiments, the ligand is selected from the group consisting of cholesterol, biotin, vitamins, galactose derivatives or analogs, lactose derivatives or analogs, N-acetylgalactosamine derivatives or analogs, and N-acetylglucosamine derivatives or analogs. The ligand targets a cell surface receptor, including galactose, galactosamine, lactose, or N-acetylgalactosamine/glucosamine moieties. The ligand preferably targets the liver, in particular, the parenchymal cells of the liver.

In some preferred embodiments, the ligand targets an ASGPR receptor.

In some preferred embodiments, the ligand may also be human serum albumin (HSA), hyaluronic acid, or a polypeptide, etc.

In some preferred embodiments, the ligand is L96, having a structure and a conjugating to the sense strand as follows.

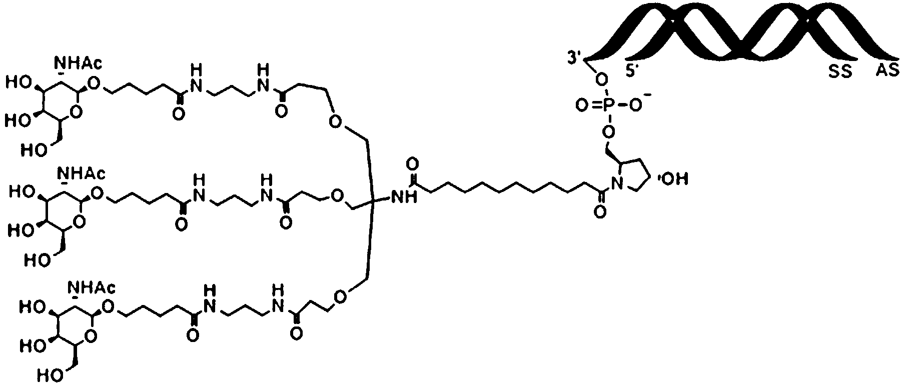

In some preferred embodiments, a dsRNA molecule comprising a ligand modification is selected from the group consisting of:
GAL-E5, comprising a sense strand of SEQ ID NO: 5 comprising the modifications as defined in Modification Motif i and L96 conjugated to the 3' end, and an antisense strand of SEQ ID NO: 105 comprising the modifications as defined in Modification Motif iv;
GAL-E17, comprising a sense strand of SEQ ID NO: 17 comprising the modifications as defined in Modification Motif i and L96 conjugated to the 3' end, and an antisense strand of SEQ ID NO: 117 comprising the modifications as defined in Modification Motif iv;
GAL-E85-1, comprising a sense strand of SEQ ID NO: 85 comprising the modifications as defined in Modification Motif i and L96 conjugated to the 3' end, and an antisense strand of SEQ ID NO: 185 comprising the modifications as defined in Modification Motif iii; and
GAL-E85, comprising a sense strand of SEQ ID NO: 85 comprising the modifications as defined in Modification Motif i and L96 conjugated to the 3' end, and an antisense strand of SEQ ID NO: 185 comprising the modifications as defined in Modification Motif iv.

For above, as described herein, each Modification Motif is as follows.

Modification Motif i:
XmsXmsXmXmXmXmXfXmXfXfXfXmXmXmXmXmXmXmXmXmXm;

Modification Motif ii:
XmsXmsXfXmXfXmXfXmXfXfXfXmXfXmXmXfXmXfXmXmXm;

Modification Motif iii:
XmsXfsXmXmXmXfXmXmXmXmXmXmXmXfXmXfXmXmXmXmXmsXmsXm;

Modification Motif iv:
XmsXfsXmXmXmXmXmXmXmXmXmXmXmXfXmXfXmXmXmXmXmsXmsXm; and

Modification Motif v:
XmsXfsXfXmXfXmXmXfXmXfXmXmXmXfXmXfXmXfXmXmXmsXmsXmdTdT;
wherein "Xm" represents a ribonucleotide comprising a 2'-O-methyl modification, "Xf" represents a ribonucleotide comprising a 2'-fluoro modification, and "s" represents a phosphorothioate backbone linkage between two flanking ribonucleotides.

It should be understood that, in the present disclosure, for a sense strand of a dsRNA molecule comprising the modifications as defined in Modification Motif i or ii, or an antisense strand of a dsRNA molecule comprising the modification as defined in Modification Motif iii, iv or v, "X's" in each Modification Motif represent the ribonucleotides of the sense strand or the antisense strand from the 5' end to 3' end in sequence, in which the ribonucleotides of the sense strand or the antisense strand from the 5' end to 3' end have the same ribonucleotide modifications as individual "X" at the same position in the corresponding Modification Motif. For example, for a sense strand of a dsRNA molecule comprising the modification as defined in Modification Motif i, the ribonucleotides of the sense strand of the dsRNA molecule from the 5' end to 3' end have the same ribonucleotide modifications as individual "X's" in Modification Motif i from the 5' end to 3' end in sequence. That is, the sense strand has 2'-fluoro modifications at positions 7 and 9 to 11 from the 5' end and 2'-O-methyl modifications at the remaining positions, and has phosphorothioate backbone linkages among the contiguous nucleotides at positions 1 to 3 from the 5' end. Similarly, when for an antisense strand of a dsRNA molecule comprising the modification as defined in Modification Motif iv, the ribonucleotides of the antisense strand of the dsRNA molecule from the 5' end to 3' end have the same ribonucleotide modifications as individual "X's" in Modification Motif iv from the 5' end to 3' end in sequence. That is, the antisense strand has 2'-fluoro modifications at positions 2, 14 and 16 from the 5' end and 2'-O-methyl modifications at the remaining positions, and has phosphorothioate backbone linkages among the contiguous nucleotides at positions 1 to 3 from the 5' end and among the contiguous nucleotides at positions 1 to 3 from the 3' end. In some embodiments, for a sense strand of a dsRNA molecule comprising the modification as defined in Modification Motif i or ii, or an antisense strand of a dsRNA molecule comprising the modification as defined in Modification Motif iii, iv or v, the nucleotides at individual positions of the dsRNA molecule do not contain other chemical modifications except for the nucleotide modifications defined for corresponding "X's" in the Modification Motifs. In some embodiments, for a sense strand of a dsRNA molecule comprising the modification as defined in Modification Motif i or ii, or an antisense strand of a dsRNA molecule comprising the modification as defined in Modification Motif iii, iv or v, the nucleotides at individual positions of the dsRNA molecule do not contain other chemical modifications except for the nucleotide modifications defined for corresponding "X's" in the Modification Motifs and ligand conjugations to the 3' end of the sense strand.

In some more preferred embodiments, a dsRNA molecule comprising a ligand modification is:
a dsRNA molecule, comprising a sense strand of SEQ ID NO: 5 comprising the modifications as defined in Modification Motif i and L96 conjugated to the 3' end, and an antisense strand of SEQ ID NO: 105 comprising the modifications as defined in Modification Motif iv; or
a dsRNA molecule, comprising a sense strand of SEQ ID NO: 85 comprising the modifications as defined in Modification Motif i and L96 conjugated to the 3' end, and an antisense strand of SEQ ID NO: 185 comprising the modifications as defined in Modification Motif iv.

In some preferred embodiments, nucleotides comprising modifications account for 0% to 100% of individual strands of the dsRNA molecules, such as 5%, 10%, 15%, 20%, 25%, 30%, 35%, 40%, 45%, 50%, 55%, 60%, 65%, 70%, 75%, 80%, 85%, 90%, 95%, 99% or 100%. The modifications can be in the overhang regions or in the double-stranded regions. The modifications can be used to improve *in vitro* or *in vivo* characteristics of the dsRNA molecules, such as stability, biodistribution, inhibitory activity, etc. The above modifications can be used in combination.

In some preferred embodiments, individual strands of the dsRNA molecules have overhangs or blunt ends. Overhangs include 1 to 8 nucleotides at the 5' and/or 3' end(s) of one or both strand(s), such as 1, 2, 3, 4, 5, 6, 7, or 8 nucleotides, and the nucleotides in the overhangs can be any one selected from the group consisting of U, A, G, C, T, and dT.

In some preferred embodiments, the dsRNA molecule is capable of inhibiting the gene expression of C5 in human or cynomolgus monkey.

In another aspect, the present invention relates to biological materials associated with the dsRNA.

In some embodiments, the dsRNA-associated biological material is selected from the group consisting of:
(A) a DNA molecule capable of producing the dsRNA;
(B) a vector capable of expressing the dsRNA;
(C) an agent or a kit comprising the dsRNA, the DNA molecule, or the vector; and
(D) a pharmaceutical composition comprising the dsRNA molecule and other pharmaceutically acceptable components.

In some embodiments, the pharmaceutical composition comprises a pharmacologically effective amount of the dsRNA molecule of the present invention and other pharmaceutically acceptable components. The term "effective amount" refers to the amount of the dsRNA molecule that can effectively produce desired pharmacological or therapeutic effects.

In some embodiments, "other components" include water, saline, glucose, buffers such as PBS, excipients, diluents, disintegrants, binders, lubricants, sweeteners, flavoring agents, preservatives, or a combination thereof.

In another aspect, the present invention relates to the use of the dsRNA or related biological materials for preventing and/or treating diseases mediated by C5 gene, or for alleviating the symptoms of diseases mediated by C5 gene. In yet another aspect, the present invention relates to the use of the dsRNA or related biological materials for manufacturing a formulation or a medicament for preventing and/or treating diseases mediated by C5 gene, or for alleviating the symptoms of diseases mediated by C5 gene. The diseases mediated by C5 gene include, but are not limited to, paroxysmal nocturnal hemoglobinuria (PNH), atypical hemolytic uremic syndrome (aHUS), asthma, rheumatoid arthritis (RA), antiphospholipid antibody syndrome, lupus nephritis, ischemia-reperfusion injury, typical or infectious hemolytic uremic syndrome (tHUS), dense deposit disease (DDD), neuromyelitis optica (NMO), multifocal motor neuropathy (MMN), multiple sclerosis (MS), macular degeneration such as age-related macular degeneration (AMD), hemolysis, elevated liver enzymes and low platelets (HELLP) syndrome, thrombotic thrombocytopenic purpura (TTP), spontaneous abortion, pauci-immune vasculitis, epidermolysis bullosa, habitual abortion, preeclampsia, traumatic brain injury, myasthenia gravis, cold agglutinin disease, dermatomyositis, bullous pemphigoid, Shiga toxin-producing *Escherichia coli* (*E. coli*)-associated hemolytic uremic syndrome, C3 nephropathy, antineutrophil cytoplasmic antibodyassociated vasculitis, humoral and vascular transplant rejection, graft dysfunction, myocardial infarction, allogeneic transplantation, sepsis, coronary artery disease, dermatomyositis, Graves' disease, atherosclerosis, Alzheimer's disease, systemic inflammatory response sepsis, septic shock, spinal cord injury, glomerulonephritis, Hashimoto's thyroiditis, type I diabetes, psoriasis, pemphigus, autoimmune hemolytic anemia (AIHA), ITP, Goodpasture's syndrome, Degos' disease, antiphospholipid syndrome (APS), catastrophic APS (CAPS), cardiovascular disorders, myocarditis, cerebrovascular disease, peripheral vascular disease, renovascular disease, mesenteric/intestinal vascular disease, vasculitis, Henoch-Schönlein purpura nephritis, vasculitis associated with systemic lupus erythematosus, vasculitis associated with rheumatoid arthritis, immune complex vasculitis, Takayasu's disease, dilated cardiomyopathy, diabetic vasculopathy, Kawasaki's disease (arteritis), diseases associated with viral infections such as COVID-19, chronic obstructive pulmonary disease (COPD), acute respiratory distress syndrome (ARDS), C5-related tumors such as liver cancer or lung cancer, venous gas embolism (VGE) and stent placement, rotational atherectomy, membranous nephropathy, Guillain-Barré syndrome, and restenosis after percutaneous transluminal coronary angioplasty (PTCA).

In yet another aspect, the present invention relates to the use of dsRNA or related biological materials for manufacturing a medicament for preventing and/or treating IgA nephropathy.

In some embodiments, the present invention further provides any of the following applications.

The present invention provides the use of the dsRNA or the biological material for inhibiting the gene expression of C5, or manufacturing a product for inhibiting the gene expression of C5.

In the above use, "inhibiting the gene expression of C5" refers to inhibiting the gene expression of human or monkey C5 or reducing the gene expression level of human or monkey C5 in cells *in vivo* or *in vitro,* and means that the gene expression level of C5 is inhibited or reduced by at least 95%, 90%, 85%, 80%, 75%, 70%, 60%, 50%, 40%, 30%, 20%, 10%, or 5%. Detection of the level of a target gene, RNA or protein can be used to predict or assess the activity, efficacy or therapeutic outcome.

In some embodiments, the cells are mammalian cells expressing C5 gene, such as primate cells or human cells. Preferably, C5 gene is expressed at a high level in the target cells. More preferably, the cells are derived from the brain, lung, liver, kidney, or tumor. Even more preferably, the cells are liver cancer cells.

In some embodiments, the cells are selected from the group consisting of HepG2 cells, HEP3B cells, Huh7 cells, MHCC97H cells, Hela cells, primary cells derived from cynomolgus monkey, and human primary cells.

In some embodiments, the final cellular concentration of the dsRNA molecule is 0.001 to 1000 nM, such as 0.001 to 10 nM, 10 to 500 nM, 25 to 300 nM, or 50 to 100 nM.

In some embodiments, the dsRNA or related biological materials can be administered by any suitable means, such as parenteral administration, including intramuscular, intravenous, intraarterial, intraperitoneal, or subcutaneous injection; and by administration modes including but not limited to single administration or multiple administrations.

In some preferred embodiments, the dosage range is from 0.1 mg/kg to 100 mg/kg, from 0.5 mg/kg to 50 mg/kg, from 3 mg/kg to 36 mg/kg, from 2.5 mg/kg to 20 mg/kg, or from 5 mg/kg to 15 mg/kg, such as 3 mg/kg, 4 mg/kg, 5 mg/kg, 6 mg/kg, 7 mg/kg, 8 mg/kg, 9 mg/kg, 10 mg/kg, 11 mg/kg, 12 mg/kg, 13 mg/kg g, 14mg/kg, 15mg/kg, 16mg/kg, 17mg/kg, 18mg/kg, 19mg/kg, 20mg/kg, 21mg/kg, 22mg/kg, 23mg/kg, 24mg/kg, 25m g/kg, 26mg/kg, 27mg/kg, 28mg/kg, 29mg/kg, 30mg/kg, 31mg/kg, 32mg/kg, 33mg/kg, 34mg/kg, 35mg/kg, or 36mg/kg.

In some embodiments, a single dose of the pharmaceutical composition can be long-lasting, with the reduction in the gene expression level of C5 lasting for at least 3, 5, 7, 10, or 14 days or longer.

In some embodiments, the present invention provides the use of the dsRNA or biological material for reducing C5 in a serum, or manufacturing a product for reducing C5 in a serum.

In the above use, "reducing C5 in a serum" refers to the reduction of C5 concentration in a serum in human or monkey. For example, serum C5 concentration or content is reduced by at least 5%, 10%, 15%, 20%, 25%, 30%, 35%, 40%, 50%, 60%, 70%, 80%, 90%, 95%, or 98%.

In some embodiments, the present invention provides the use of the dsRNA or biological material for preventing and/or treating a disease mediated by C5 gene, or manufacturing a product for preventing and/or treating a disease mediated by C5 gene.

In some embodiments, the present invention provides the use of the dsRNA or biological material for alleviating the symptoms of a disease mediated by C5 gene, or manufacturing a product for alleviating the symptoms of a disease mediated by C5 gene.

In some embodiments, the disease mediated by C5 gene is preferably an immune-related disease, a hematology-related disease, or a metabolism-related disease.

In some embodiments, the disease mediated by C5 gene or symptoms thereof may be caused by overexpression of C5 gene or overproduction of C5 protein, which can be regulated by downregulating the gene expression of C5. In the above use, "treating" refers to alleviating, reducing or curing of the disease mediated by C5 gene or symptoms thereof, such as reducing C5 level in a serum. For example, serum C5 content or concentration can be reduced by 5%, 10%, 15%, 20%, 25%, 30%, 35%, 40%, 50%, 60%, 70%, 80%, 90%, or 95%.

In some embodiments, the present invention further provides the use of the dsRNA molecules comprising ligand modifications in the preparation of a liver-targeted drug. The liver-targeted drug can be used to treat liver diseases mediated by C5 gene.

In another aspect, the present invention further provides a method and/or combination therapy for treating a subject suffering from a disease that would benefit from inhibition or reduction of C5 gene expression, such as a complement component C5-associated disease, for example, paroxysmal nocturnal hemoglobinuria (PNH) or atypical hemolytic uremic syndrome (aHUS), using an RNAi composition capable of acting on RNA-induced silencing complex (RISC)-mediated cleavage of the RNA transcripts of C5 gene.

The combination therapies of the present invention comprise administering to a patient suffering from a complement component C5-associated disease an RNAi agent of the present invention and an additional therapeutic agent, such as an anti-complement component C5 antibody or antigen-binding fragment thereof, e.g., eculizumab. These combination therapies of the present invention can reduce C5 level in a subject (e.g., by about 30%, 35%, 40%, 45%, 50%, 55%, 60%, 65%, 70%, 75%, 80%, 85%, 90%, 95%, or about 99%) by targeting C5 mRNA with an RNAi agent of the present invention, thereby allowing a reduction in the therapeutically (or prophylactically) effective amount of eculizumab required to treat the subject, and thus reducing treatment costs and allowing simpler and more convenient methods of administering eculizumab, such as subcutaneous administration.

In some embodiments, the additional therapeutic agent may be an anti-complement component C5 antibody or an antigen-binding fragment or derivative thereof.

The innovation of the present invention lies in the following aspects.
i) The dsRNA molecules comprising modifications exhibit high stability and inhibitory activity.
ii) The dsRNA molecules comprising ligand modifications, in addition to maintaining high inhibitory activity and stability, possess good liver-targeting properties and the ability to facilitate cell endocytosis, which can reduce the impact on other tissues or organs and lower the required amount of dsRNA molecules, thereby achieving the goal of reducing toxicity and costs. In addition, the dsRNA molecules comprising ligand modifications can enter target cells and tissues without a transfection agent, thereby reducing the negative effects associated with the transfection agent, such as cell or tissue toxicity, and thus enabling targeted therapy. Although many modifications can be attempted to improve the performance of dsRNA, achieving both mediating RNA interference and improving stability in a serum (e.g., exhibiting increased resistance to nucleases and/or prolonged duration of action) remains challenging. The modified dsRNAs of the present invention exhibit high stability while retaining high inhibitory activity, achieving unexpected technical effects.

### Definition

In siRNAs, "an antisense strand (AS)" refers to one strand that can complementarily pair with the mRNA of a target gene, and "a sense strand (SS)" refers to the other strand.

"G", "C", "A", "T", and "U' generally represent nucleotides containing guanine, cytosine, adenine, thymine, and uracil as their respective bases.

"Relative expression level (REL)" refers to relative mRNA expression level.

"GalNAc" refers to N-acetylgalactosamine.

In the context of modifications, "N" refers to RNA; "dN" refers to DNA; "Nm" refers to 2'-OMe modification; "Nf" refers to 2'-F modification; and "(s)" refers to a PS backbone, i.e., 5'-thio-modified phosphate backbone.

"Complement component C5" is used interchangeably with the term "C5", and refers to the well-known gene and polypeptide, which is also known in the art as Prepro-C5, ECLZB, CPAMD4, C3 and PZP-like alpha-2-macroglobulin domain-containing protein, anaphylatoxin C5a analog, hemolytic complement (Hc) and complement C5. The sequence of human C5 mRNA transcript can be found, for example, in GenBank Accession No. GI: 38016946 (NM_001735.2; SEQ ID NO: 207). The sequence of cynomolgus monkey C5 mRNA can be found, for example, in GenBank Accession No. GI: 297270262 (XM_001095750.2; SEQ ID NO: 208). The sequence of mouse C5 mRNA can be found, for example, in GenBank Accession No. GI: 291575171 (NM_010406.2; SEQ ID NO: 209). The sequence of rat C5 mRNA can be found, for example, in GenBank Accession No. GI: 392346248 (XM_345342.4; SEQ ID NO:210). Additional examples of C5 mRNA sequences are readily obtained using publicly available databases such as GenBank.

Generally, most nucleotides in each strand of the dsRNA molecule are ribonucleotides, but as described in detail herein, one strand or both strands may also include one or more nonribonucleotide nucleotides, such as deoxyribonucleotides and/or modified nucleotides. In addition, as used herein, An "RNAi agent" may include ribonucleotides comprising chemical modifications and may contain multiple modifications on multiple nucleotides. Such modifications may include all types of modifications disclosed herein or known in the art. Any such modifications as used in siRNA-type molecules are intended to be encompassed by an "RNAi agent" for purposes of the present specification and appended claims.

### BRIEF DESCRIPTION OF THE DRAWINGS

FIG. 1 shows the results from high-throughput screening of dsRNA molecules.
FIG. 2 shows the results from single-dose screening of unmodified candidate dsRNA molecules in HepG2 cells.
FIG. 3 shows the results from single-dose screening of modified dsRNA molecules in HepG2 cells.
FIG. 4 shows the IC₅₀ results of modified dsRNAs in HepG2 cells.
FIG. 5 shows the results from single-dose screening of GalNAc-conjugated dsRNAs in HepG2 cells.
FIG. 6 shows the results from single-dose screening of GalNAc-conjugated dsRNA in primary hepatocytes derived from cynomolgus monkey.
FIG. 7 shows the results of C5 content measurement after a single 1 mpk administration *in vivo.*
FIG. 8 shows the results of C5 content measurement after a gradient single-dose administration *in vivo.*
FIG. 9 shows the results of C5 content measurement after a single administration in cynomolgus monkey.
FIG. 10 shows the comparison of antisense strands in two Modification Motifs (Modification Motif iii vs. Modification Motif iv).

### Examples

### Example 1: Activity Screening Assay for C5-dsRNAs

### 1. Design of dsRNAs

Based on the human C5 mRNA sequence, different sites were selected to design multiple C5 dsRNAs. All designed individual dsRNAs can target all transcripts of the target gene as shown in Table 1. The sequences as set forth in Table 2 have minimal homology with all other non-target gene sequences after sequence alignment using a similarity software.

In the present invention, the sequence of C5 dsRNA product, Cemdisiran (patent sequence AD-62643), which is under clinical development by Alnylam Pharmaceuticals, is used as the positive reference. Based on the status of the relevant sequences (non-modified or modified, conjugated ligand, etc.), these sequences are named PC, PC-ESC or GAL-PC in the present invention.

**Table 1. Target genes**

| Target gene | Species | Gene ID | NM_ID |
|---|---|---|---|
| C5 | *Homo sapiens* (human) | 727 | NM_001735.3 |
| | | | SEQ ID NO: 211 |
| C5 | *Macaca fascicularis* (cynomolgus monkey) | 102125658 | XM_005580915.3 |
| | | | SEQ ID NO: 212 |

**Table 2. dsRNA sequences for high-throughput screening**

| No. | Sequence of sense strand (5' to 3') | SEQ ID NO: | Sequence of anti-sense strand (5' to 3') | SEQ ID NO: |
|---|---|---|---|---|
| 1 | AUUUCAGCACCAAAAAUAUUC | 1 | GAAUAUUUUUGGUGCUGAAAUGA | 101 |
| 2 | UUCAGCACCAAAAAUAUUCCG | 2 | CGGAAUAUUUUUGGUGCUGAAAU | 102 |
| 3 | UCAGCACCAAAAAUAUUCCGU | 3 | ACGGAAUAUUUUUGGUGCUGAAA | 103 |
| 4 | UAUUGUGAUUCAAGUUUAUGG | 4 | CCAUAAACUUGAAUCACAAUAUU | 104 |
| 5 | GUGAUUCAAGUUUAUGGAUAC | 5 | GUAUCCAUAAACUUGAAUCACAA | 105 |
| 6 | GAUUCAAGUUUAUGGAUACAC | 6 | GUGUAUCCAUAAACUUGAAUCAC | 106 |
| 7 | AGCAUUUGAUGCAACAAUCUC | 7 | GAGAUUGUUGCAUCAAAUGCUUC | 107 |
| 8 | GCAACAAUCUCUAUUAAAAGU | 8 | ACUUUUAAUAGAGAUUGUUGCAU | 108 |
| 9 | CAACAAUCUCUAUUAAAAGUU | 9 | AACUUUUAAUAGAGAUUGUUGCA | 109 |
| 10 | UCUCUAUUAAAAGUUAUCCUG | 10 | CAGGAUAACUUUUAAUAGAGAUU | 110 |
| 11 | AGUUAUCCUGAUAAAAAAUUU | 11 | AAAUUUUUUAUCAGGAUAACUUU | 111 |
| 12 | AUUUAUCCUCAGAGAAUAAAU | 12 | AUUUAUUCUCUGAGGAUAAAUGA | 112 |
| 13 | UUAUCCUCAGAGAAUAAAUUC | 13 | GAAUUUAUUCUCUGAGGAUAAAU | 113 |
| 14 | GAGAAUAAAUUCCAAAACUCU | 14 | AGAGUUUUGGAAUUUAUUCUCUG | 114 |
| 15 | UCUUAACAAUACAACCAAAAC | 15 | GUUUUGGUUGUAUUGUUAAGAUU | 115 |
| 16 | CCCAGUUUCUUAUGUGUAUUU | 16 | AAAUACACAUAAGAAACUGGGUU | 116 |
| 17 | UGUGUAUUUGGAAGUUGUAUC | 17 | GAUACAACUUCCAAAUACACAUA | 117 |
| 18 | AGUUGUAUCAAAGCAUUUUUC | 18 | GAAAAAUGCUUUGAUACAACUUC | 118 |
| 19 | CUUCAUUCAUACAGACAAACC | 19 | GGUUUGUCUGUAUGAAUGAAGAG | 119 |
| 20 | ACAGACAAACCUGUUUAUACU | 20 | AGUAUAAACAGGUUUGUCUGUAU | 120 |
| 21 | CAGACAAACCUGUUUAUACUC | 21 | GAGUAUAAACAGGUUUGUCUGUA | 121 |
| 22 | AACCUGUUUAUACUCCAGACC | 22 | GGUCUGGAGUAUAAACAGGUUUG | 122 |
| 23 | AGAGUUUAUUCGUUGAAUGAC | 23 | GUCAUUCAACGAAUAAACUCUAA | 123 |
| 24 | CUGUCUUAACUUUCAUAGAUC | 24 | GAUCUAUGAAAGUUAAGACAGUU | 124 |
| 25 | GUCUUAACUUUCAUAGAUCCU | 25 | AGGAUCUAUGAAAGUUAAGACAG | 125 |
| 26 | UUGAUCAUAUUGGAAUUAUCU | 26 | AGAUAAUUCCAAUAUGAUCAAUU | 126 |
| 27 | UGAUCAUAUUGGAAUUAUCUC | 27 | GAGAUAAUUCCAAUAUGAUCAAU | 127 |
| 28 | UCAUAUUGGAAUUAUCUCUUU | 28 | AAAGAGAUAAUUCCAAUAUGAUC | 128 |
| 29 | UUUUCCUGACUUCAAGAUUCC | 29 | GGAAUCUUGAAGUCAGGAAAAGA | 129 |
| 30 | ACUUCAAGAUUCCGUCUAAUC | 30 | GAUUAGACGGAAUCUUGAAGUCA | 130 |
| 31 | CUUCAAGAUUCCGUCUAAUCC | 31 | GGAUUAGACGGAAUCUUGAAGUC | 131 |
| 32 | UUCCGUCUAAUCCUAGAUAUG | 32 | CAUAUCUAGGAUUAGACGGAAUC | 132 |
| 33 | UAAUCCUAGAUAUGGUAUGUG | 33 | CACAUACCAUAUCUAGGAUUAGA | 133 |
| 34 | UAAAUAUAAAGAGGACUUUUC | 34 | GAAAAGUCCUCUUUAUAUUUAGC | 134 |
| 35 | AUUACUAUAAAAGCAAGAUAU | 35 | AUAUCUUGCUUUUAUAGUAAUUU | 135 |
| 36 | AAGCAAGAUAUUUUUAUAAUA | 36 | UAUUAUAAAAAUAUCUUGCUUUU | 136 |
| 37 | UUUUAUAAUAAAGUAGUCACU | 37 | AGUGACUACUUUAUUAUAAAAAU | 137 |
| 38 | CACUGAGGCUGACGUUUAUAU | 38 | AUAUAAACGUCAGCCUCAGUGAC | 138 |
| 39 | ACUGAGGCUGACGUUUAUAUC | 39 | GAUAUAAACGUCAGCCUCAGUGA | 139 |
| 40 | AAAGAUGAUCAAAAAGAAAUG | 40 | CAUUUCUUUUUGAUCAUCUUUUA | 140 |
| 41 | AAAACACAAUGUUGAUAAAUG | 41 | CAUUUAUCAACAUUGUGUUUUGC | 141 |
| 42 | AAACACAAUGUUGAUAAAUGG | 42 | CCAUUUAUCAACAUUGUGUUUUG | 142 |
| 43 | UGUCAUACUACAGUUUAGAAG | 43 | CUUCUAAACUGUAGUAUGACAGU | 143 |
| 44 | AAGAUUUAAACAACAAGUACC | 44 | GGUACUUGUUGUUUAAAUCUUCU | 144 |
| 45 | GAUUUAAACAACAAGUACCUU | 45 | AAGGUACUUGUUGUUUAAAUCUU | 145 |
| 46 | AAACAACAAGUACCUUUAUAU | 46 | AUAUAAAGGUACUUGUUGUUUAA | 146 |
| 47 | AACAACAAGUACCUUUAUAUU | 47 | AAUAUAAAGGUACUUGUUGUUUA | 147 |
| 48 | ACAACAAGUACCUUUAUAUUG | 48 | CAAUAUAAAGGUACUUGUUGUUU | 148 |
| 49 | CAACAAGUACCUUUAUAUUGC | 49 | GCAAUAUAAAGGUACUUGUUGUU | 149 |
| 50 | CUUUAUAUUGCUGUAACAGUC | 50 | GACUGUUACAGCAAUAUAAAGGU | 150 |
| 51 | CAGAAAUACCUGGCAUCAAAU | 51 | AUUUGAUGCCAGGUAUUUCUGCC | 151 |
| 52 | CAGGUUAAAGAUUCGCUUGAC | 52 | GUCAAGCGAAUCUUUAACCUGCA | 152 |
| 53 | AGGUUAAAGAUUCGCUUGACC | 53 | GGUCAAGCGAAUCUUUAACCUGC | 153 |
| 54 | ACGGUGCUGGAGUUUAAUGUC | 54 | GACAUUAAACUCCAGCACCGUCA | 154 |
| 55 | CUGGAGUUUAAUGUCAAAACU | 55 | AGUUUUGACAUUAAACUCCAGCA | 155 |
| 56 | UGGAGUUUAAUGUCAAAACUG | 56 | CAGUUUUGACAUUAAACUCCAGC | 156 |
| 57 | GGGAAGGUUACCGAGCAAUAG | 57 | CUAUUGCUCGGUAACCUUCCCUG | 157 |
| 58 | GGAAGGUUACCGAGCAAUAGC | 58 | GCUAUUGCUCGGUAACCUUCCCU | 158 |
| 59 | CCGAGCAAUAGCAUACUCAUC | 59 | GAUGAGUAUGCUAUUGCUCGGUA | 159 |
| 60 | AUAUUGAUUGGACUGAUAACC | 60 | GGUUAUCAGUCCAAUCAAUAUAA | 160 |
| 61 | AUUAUUGUUACCCCCAAAAGC | 61 | GCUUUUGGGGGUAACAAUAAUAU | 161 |
| 62 | CCCAAAAGCCCAUAUAUUGAC | 62 | GUCAAUAUAUGGGCUUUUGGGGG | 162 |
| 63 | CCAUAUAUUGACAAAAUAACU | 63 | AGUUAUUUUGUCAAUAUAUGGGC | 163 |
| 64 | CAUAUAUUGACAAAAUAACUC | 64 | GAGUUAUUUUGUCAAUAUAUGGG | 164 |
| 65 | GACAAAAUAACUCACUAUAAU | 65 | AUUAUAGUGAGUUAUUUUGUCAA | 165 |
| 66 | ACAAAAUAACUCACUAUAAUU | 66 | AAUUAUAGUGAGUUAUUUUGUCA | 166 |
| 67 | CAAAAUAACUCACUAUAAUUA | 67 | UAAUUAUAGUGAGUUAUUUUGUC | 167 |
| 68 | AACUCACUAUAAUUACUUGAU | 68 | AUCAAGUAAUUAUAGUGAGUUAU | 168 |
| 69 | UUUAUCCAAGGGCAAAAUUAU | 69 | AUAAUUUUGCCCUUGGAUAAAAU | 169 |
| 70 | UUAUCCAAGGGCAAAAUUAUC | 70 | GAUAAUUUUGCCCUUGGAUAAAA | 170 |
| 71 | UAUCCAAGGGCAAAAUUAUCC | 71 | GGAUAAUUUUGCCCUUGGAUAAA | 171 |
| 72 | GGGCAAAAUUAUCCACUUUGG | 72 | CCAAAGUGGAUAAUUUUGCCCUU | 172 |
| 73 | GGCAAAAUUAUCCACUUUGGC | 73 | GCCAAAGUGGAUAAUUUUGCCCU | 173 |
| 74 | AAUUUUCAGAUGCAUCUUAUC | 74 | GAUAAGAUGCAUCUGAAAAUUUC | 174 |
| 75 | AUCUUAUCAAAGUAUAAACAU | 75 | AUGUUUAUACUUUGAUAAGAUGC | 175 |
| 76 | UCUUAUCAAAGUAUAAACAUU | 76 | AAUGUUUAUACUUUGAUAAGAUG | 176 |
| 77 | CUUAUCAAAGUAUAAACAUUC | 77 | GAAUGUUUAUACUUUGAUAAGAU | 177 |
| 78 | UGAUUCAGUCUGGUUAAAUAU | 78 | AUAUUUAACCAGACUGAAUCAGA | 178 |
| 79 | GAUUCAGUCUGGUUAAAUAUU | 79 | AAUAUUUAACCAGACUGAAUCAG | 179 |
| 80 | UUAAAUAUUGAAGAAAAAUGU | 80 | ACAUUUUUCUUCAAUAUUUAACC | 180 |
| 81 | AACUGUGUCUCUUAAUAUGGC | 81 | GCCAUAUUAAGAGACACAGUUUG | 181 |
| 82 | UUGGAAAGAGUAUUUCAAUUC | 82 | GAAUUGAAAUACUCUUUCCAAGG | 182 |
| 83 | AAAAGAAGAUAGAAGAAAUAG | 83 | CUAUUUCUUCUAUCUUCUUUUGC | 183 |
| 84 | AAAUAGCUGCUAAAUAUAAAC | 84 | GUUUAUAUUUAGCAGCUAUUUCU | 184 |
| 85 | GUGAAGAAAUGUUGUUACGAU | 85 | AUCGUAACAACAUUUCUUCACUA | 185 |
| 86 | UGAAGAAAUGUUGUUACGAUG | 86 | CAUCGUAACAACAUUUCUUCACU | 186 |
| 87 | UUCACUGAAUGUUGUGUCGUC | 87 | GACGACACAACAUUCAGUGAAAG | 187 |
| 88 | GUGCUAAUAUCUCUCAUAAAG | 88 | CUUUAUGAGAGAUAUUAGCACGG | 188 |
| 89 | AAGACCCUGUUACCAGUAAGC | 89 | GCUUACUGGUAACAGGGUCUUCA | 189 |
| 90 | GCCAGAAAUUCGGAGUUAUUU | 90 | AAAUAACUCCGAAUUUCUGGCUU | 190 |
| 91 | CCAGAAAUUCGGAGUUAUUUU | 91 | AAAAUAACUCCGAAUUUCUGGCU | 191 |
| 92 | AGAAAUUCGGAGUUAUUUUCC | 92 | GGAAAAUAACUCCGAAUUUCUGG | 192 |
| 93 | AAGAAAACAGUUGCAGUUUGC | 93 | GCAAACUGCAACUGUUUUCUUCU | 193 |
| 94 | UUUCAAACACUGGUAUAUGUG | 94 | CACAUAUACCAGUGUUUGAAAUG | 194 |
| 95 | CUUCCUGGAAAUGAAUAUACC | 95 | GGUAUAUUCAUUUCCAGGAAGAC | 195 |
| 96 | AACUGUUUACAACUAUAGGAC | 96 | GUCCUAUAGUUGUAAACAGUUCC | 196 |
| 97 | ACUGUUUACAACUAUAGGACU | 97 | AGUCCUAUAGUUGUAAACAGUUC | 197 |
| 98 | CUGUUUACAACUAUAGGACUU | 98 | AAGUCCUAUAGUUGUAAACAGUU | 198 |
| PC | AAGCAAGAUAUUUUUAUAAUA | 99 | UAUUAUAAAAAUAUCUUGCUUUU TT | 100 |

### 2. Synthesis and purification of dsRNAs (conjugates)

The dsRNAs in the present invention contain only ribonucleotides or 2'-methoxy or 2'-fluoro modified oligonucleotides, which are synthesized according with a theoretical yield of 1 µmol. For synthesis, 1 µmol of a universal Frit support (1000Å =100nm, Biocomma) or a CPG support of GalNAc derivative L96 with a protecting group (Asymchem) was selected and used to prepare all oligonucleotides on a LK-192X synthesizer. According to the sequence requirements, all nucleoside-corresponding phosphoramidite monomers were diluted with anhydrous acetonitrile solvent at a ratio of 1:40 (g/mL), and the conjugating reaction was performed twice, each for 3 min. Deprotection was performed using 3% TCA, activation was performed using 5-benzylthio-1H-tetrazole (0.3 M solution in acetonitrile), and capping and oxidation were performed with CAPA/CAPB and 50 mM I₂ solution, respectively. After trityl-off synthesis, the solid phase support was transferred to a 2 mL centrifuge tube, 1.2 mL of ammonium hydroxide was added, and the tube was heated in an oven at 65° C for 3 h to remove the protecting group. The solution was then cooled to room temperature, concentrated under vacuum for 30 minutes, and filtered through a 0.22µm filter membrane into a sample vial. A semi-preparative reverse phase purification instrument was used for single-strand purification with an elution gradient of 7% to 30% (ACN: 100mM TEAA) at a flow rate of 5mL/min for 10 min. After purification, the sample was vacuum-concentrated and then spin-dried at room temperature. Finally, the sample was dissolved in water, and each solution was desalted on a GE Hi-Trap desalting column to elute the final oligonucleotide product. All characteristics and purity were confirmed using ESI-MS and IEX HPLC, respectively, and the concentration was determined by UV absorbance using an ELISA reader. The final product was obtained by mixing equimolar amounts of the sense strand and antisense strand in a new tube, heating at 95°C for 5 min, slowly annealing to room temperature, and finally spin-drying at room temperature using a vacuum concentrator.

### 3. High-throughput screening and detection of C5-dsRNAs activity in vitro

### 3.1 Construction of detection plasmid

The recombinant plasmid was constructed using a psicheck-2 plasmid (GenScript Biotech), which contained the target sequences of all C5-dsRNAs to be tested, , and had cloning sites at the 5' XhoI and 3' NotI sites of the psicheck-2 plasmid.

### 3.2 Co-transfection of C5-dsRNAs and recombinant plasmid into 293T cells

All cells were purchased from ATCC; and other reagents were commercially available.

### Cell transfection

Cells were cultured in DMEM medium supplemented with 10% fetal bovine serum in a 5% CO₂, 37°C constant temperature incubator. Transfection was performed when the cells were in the logarithmic growth phase and in good condition (70% confluence). 293T human embryonic kidney cells were adjusted to a concentration of 3×10⁵/mL, and then 0.1 mL of cell solution was added to each well in a 96-well plate and cultured overnight.

### Preparation of transfection complex

Mixture A: 8 µL of Opti-MEM + 8 ng of recombinant plasmid + 2 µL of 10 nM dsRNA.
Mixture B: 9.5 µL of Opti-MEM + 0.5 µL of Lipofectamine^{®} 2000.

Each mixture was incubated for 5 min, then combined and incubated for an additional 20 min. The prepared transfection complex was added to the 96-well plate and incubated at 37°C with 5% CO₂ for 6 hours. After removing the supernatant, 0.1 mL of complete medium was added into each well and the cells were cultured for another 24 hours before sample collection. The experiments were performed in triplicate. The relative mRNA expression level in the transfection reagent-only control group (Mock group) was set as 100%, and the values in other groups were all relative expression activity or expression level relative to the Mock group.

### 3.3 DLR assay

A Dual-Luciferase^{®} Reporter Assay System kit (Promega) was used for detection. The cells were lysed and processed according to the manufacturer's instructions. The fluorescence intensity of Firefly luciferase (Photinus pyralis) and Renilla luciferase (Renilla reniformis) were measured sequentially using an Infinite Eplex microplate reader (TECAN). The Renilla/Firefly luminescence ratio was calculated and normalized to the ratio of the Mock group (set as 100%). The results of DLR assay, i.e., the average values of the dual luciferase reporter gene expression levels in C5 dsRNA test groups relative to that of the Mock group, were shown in Table 3.

**Table 3. High-throughput screening results in 293T cells**

| No. | Relative Activity % (10nM) |
|---|---|
| 1 | 10.7 |
| 2 | 53.0 |
| 3 | 41.2 |
| 4 | 4.2 |
| 5 | 7.9 |
| 6 | 24.5 |
| 7 | 5.9 |
| 8 | 10.2 |
| 9 | 16.6 |
| 10 | 27.1 |
| 11 | 13.1 |
| 12 | 14.7 |
| 13 | 59.1 |
| 14 | 17.2 |
| 15 | 21.6 |
| 16 | 16.0 |
| 17 | 10.6 |
| 18 | 17.3 |
| 19 | 22.3 |
| 20 | 41.8 |
| 21 | 33.4 |
| 22 | 27.3 |
| 23 | 14.9 |
| 24 | 19.6 |
| 25 | 82.8 |
| 26 | 23.8 |
| 27 | 41.3 |
| 28 | 31.2 |
| 29 | 42.2 |
| 30 | 19.1 |
| 31 | 21.0 |
| 32 | 11.3 |
| 33 | 35.0 |
| 34 | 21.1 |
| 35 | 14.4 |
| 36 | 17.8 |
| 37 | 26.7 |
| 38 | 15.4 |
| 39 | 13.8 |
| 40 | 13.0 |
| 41 | 12.0 |
| 42 | 13.6 |
| 43 | 16.9 |
| 44 | 11.3 |
| 45 | 35.0 |
| 46 | 21.0 |
| 47 | 17.2 |
| 48 | 21.3 |
| 49 | 21.8 |
| 50 | 18.9 |
| 51 | 14.9 |
| 52 | 18.6 |
| 53 | 20.0 |
| 54 | 53.9 |
| 55 | 163.5 |
| 56 | 141.8 |
| 57 | 88.7 |
| 58 | 50.5 |
| 59 | 13.0 |
| 60 | 160.9 |
| 61 | 42.4 |
| 62 | 28.5 |
| 63 | 37.9 |
| 64 | 34.3 |
| 65 | 25.6 |
| 66 | 28.9 |
| 67 | 27.2 |
| 68 | 45.0 |
| 69 | 25.2 |
| 70 | 44.6 |
| 71 | 49.0 |
| 72 | 31.7 |
| 73 | 30.0 |
| 74 | 29.7 |
| 75 | 60.5 |
| 76 | 59.0 |
| 77 | 50.5 |
| 78 | 24.2 |
| 79 | 39.7 |
| 80 | 97.7 |
| 81 | 38.0 |
| 82 | 40.2 |
| 83 | 32.1 |
| 84 | 12.3 |
| 85 | 5.6 |
| 86 | 21.2 |
| 87 | 23.9 |
| 88 | 24.1 |
| 89 | 41.5 |
| 90 | 23.5 |
| 91 | 27.0 |
| 92 | 26.5 |
| 93 | 13.5 |
| 94 | 71.3 |
| 95 | 28.5 |
| 96 | 24.9 |
| 97 | 17.5 |
| 98 | 18.6 |
| PC | 9.7 |

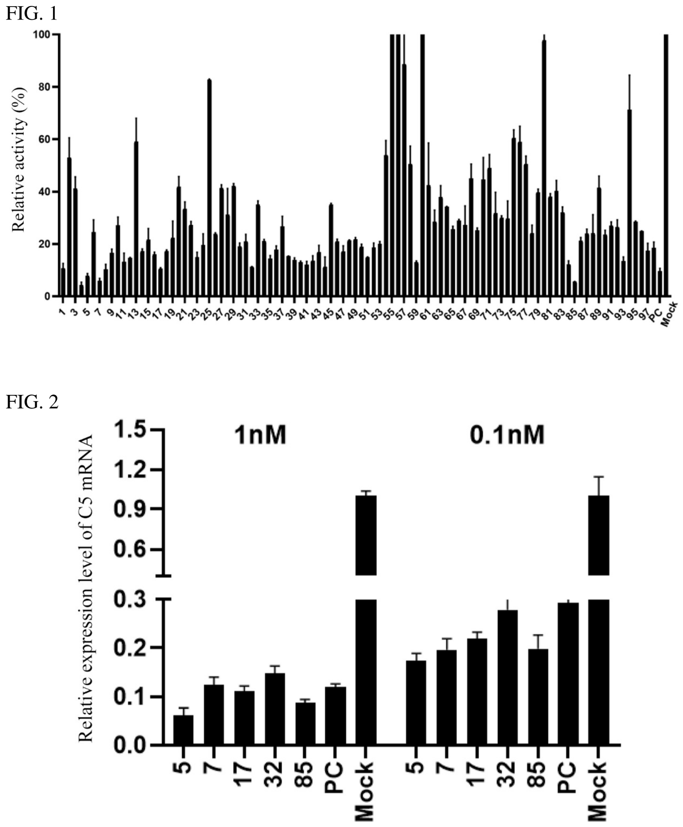

As shown in FIG. 1, multiple preferred sequences were identified through C5 dsRNA screening in 293T cells. Among these, five dsRNA molecules demonstrating superior efficacy and targeting both human and cynomolgus monkey C5 were selected as candidates, i.e., dsRNA molecule no. 5, 7, 17, 32, and 85. The unmodified positive control group (PC) also exhibited superior inhibitory activity.

### 4. Real-time quantitative PCR assay of target mRNA level

### 4.1 Transfection of C5 dsRNAs into HepG2 cells

HepG2 cells were cultured in DMEM medium supplemented with 10% fetal bovine serum in a 5% CO₂, 37°C constant temperature incubator, and plated for transfection when the cells reached the logarithmic growth phase and were in good condition (70% confluence). The cell density was adjusted to 2×10⁵ cells per well in a 24-well plate.

The transfection complex was prepared as follows. Mixture A: 250 µL of Opti-MEM + 5 µL of 10 nM or 0.1 nM dsRNA. Mixture B: 250 µL of Opti-MEM + 1.5 µL of RNAiMax transfection reagent. Each mixture was incubated for 5 min, then combined and incubated for an additional 20 min. The prepared transfection complex was added to the 24-well plate and incubated in a 5% CO₂, 37°C constant temperature incubator for 6 hours. After removing the supernatant, 1 mL of complete medium was added into each well and the cells were cultured for another 24 hours.

### 4.2 Real-time fluorescence quantitative PCR assay

The cells were lysed 24 hours post-transfection, and total cellular RNA was extracted using column-based FastPure Cell/Tissue Total RNA Isolation Kit V2 (Cat: RC112-01, Vazyme) according to Manufacturer's instructions. cDNA was synthesized via reverse transcription using Takara PrimeScrip RT Master Mix (RR036Q). QPCR primer sequences were listed in Table 3, where human GAPDH gene served as the internal reference gene. PCR reactions were performed on a CFX96 Touch Real-Time PCR Detection System (Bio-Rad, USA). The Mock group served as the normalization control, and C5 mRNA expression level in the Mock group was set as 1.

**Table 4. QPCR primer sequences**

| Name | forward primer sequence (5' to 3') | SEQ ID NO: | reverse primer sequence (5' to 3') | SEQ ID NO: |
|---|---|---|---|---|
| human-GAPDH | GATTTGGTCGTATTGGGCG C | 199 | TTCCCGTTCTCAGCCTTGAC | 200 |
| human-C5 | ACTGAATGTTGTGTCGTCG C | 201 | CCAACGCCTTGAATTTCCCA | 202 |
| mokey-C5 | CTGAAGCCTGGGATTCCAT A | 203 | CGTCACTCCAGATGGGAGAT | 204 |
| monkey-GAPDH | CGAGATCCCTCCAAAATC AA | 205 | TGACGATCTTGAGGCTGTTG | 206 |

### 4.3 Data Analysis

After PCR reactions, the Ct values of 9 replicates for one sample (3 transfection replicates x 3 qPCR replicates per sample) showed a standard deviation of ±0.5. The reference gene served for normalization. Relative quantification analysis was performed using a CFX96 software and statistical analysis was performed using a GarphPad software. Validation results for the five dsRNA molecule candidates in HepG2 cells were shown in Table 5.

**Table 5. Single-dose screening results of unmodified dsRNAs in HepG2 cells**

| No. | C5 mRNA relative expression level (1nM) | mean deviation (±SEM) | C5 mRNA relative expression level (0.1nM) | mean deviation (±SEM) |
|---|---|---|---|---|
| 5 | 0.063 | 0.014 | 0.174 | 0.015 |
| 7 | 0.124 | 0.016 | 0.174 | 0.015 |
| 17 | 0.110 | 0.012 | 0.219 | 0.014 |
| 32 | 0.148 | 0.015 | 0.279 | 0.030 |
| 85 | 0.088 | 0.007 | 0.198 | 0.028 |
| PC | 0.119 | 0.007 | 0.293 | 0.010 |

The target positions of the five dsRNA candidates (relative to human C5 transcript NM_001735.3) were positions 147-167 for No. 5, positions 173-193 for No. 7, positions 332-352 for No. 17, positions 598-618 for No. 32, and positions 2112-2132 for No. 85.

It should be understood that in the Examples of the present disclosure, all dsRNAs designated with identical nomenclature (names and numbers) possess the same naked sequences (i.e., base sequences).

### Example 2. Optimization of C5 dsRNAs

### 1. Inhibitory activity detection

To further validate the inhibitory activity of the five dsRNA molecule candidates, their sequences were subjected to modification and optimization (Table 6). A combination of fluoro and methoxy modifications were applied to different positions in the candidate sequences. The primary modification strategy is maximizing 2'-O-methyl substitutions in the antisense strand. The modification designs were shown in Table 6. HepG2 cells were transfected following the same synthesis, transfection and quantitative PCR assay procedures as described in Example 1. The average target gene expression levels, normalized to the Mock group (set as 1), were shown in Tables 7 and 8.

**Table 6. Modified dsRNA Sequences**

| Name | Naked sequence of sense strand (5' to 3') | Modification Motif | Naked sequence of antisense strand (5' to 3') | Modification Motif |
|---|---|---|---|---|
| 5-E04 | SEQ ID NO: 5 | Motif i | SEQ ID NO: 105 | Motif iii |
| 5-E05 | SEQ ID NO: 5 | Motif i | SEQ ID NO: 105 | Motif iv |
| 7-E04 | SEQ ID NO: 7 | Motif i | SEQ ID NO: 107 | Motif iii |
| 7-E05 | SEQ ID NO: 7 | Motif i | SEQ ID NO: 107 | Motif iv |
| 17-E04 | SEQ ID NO: 17 | Motif i | SEQ ID NO: 117 | Motif iii |
| 17-E05 | SEQ ID NO: 17 | Motif i | SEQ ID NO: 117 | Motif iv |
| 32-E04 | SEQ ID NO: 32 | Motif i | SEQ ID NO: 132 | Motif iii |
| 32-E05 | SEQ ID NO: 32 | Motif i | SEQ ID NO: 132 | Motif iv |
| 85-E04 | SEQ ID NO: 85 | Motif i | SEQ ID NO: 185 | Motif iii |
| 85-E05 | SEQ ID NO: 85 | Motif i | SEQ ID NO: 185 | Motif iv |
| PC-ESC | SEQ ID NO: 99 | Motif ii | SEQ ID NO: 100 | Motif v |

| | | | | |
|---|---|---|---|---|
| Note: the numbers or characters to the left of "-" in Table 6 were the name codes for the dsRNA molecules. Modification Motif i: XmsXmsXmXmXmXmXfXmXfXfXfXmXmXmXmXmXmXmXmXmXm Modification Motif ii: XmsXmsXfXmXfXmXfXmXfXfXfXmXfXmXmXfXmXfXmXmXm Modification Motif iii: XmsXfsXmXmXmXfXmXmXmXmXmXmXmXfXmXfXmXmXmXmXmsXmsXm Modification Motif iv: XmsXfsXmXmXmXmXmXmXmXmXmXmXmXfXmXfXmXmXmXmXmsXmsXm Modification Motif v: XmsXfsXfXmXfXmXmXfXmXfXmXmXmXfXmXfXmXfXmXmXmsXmsXmdTdT | | | | |

**Table 7. Single-dose screening results of modified dsRNAs in HepG2 cells**

| Name | C5 mRNA relative expression level (1nM) | standard error (±SEM) |
|---|---|---|
| 5-E04 | 0.181 | 0.012 |
| 5-E05 | 0.161 | 0.005 |
| 7-E04 | 0.705 | 0.050 |
| 7-E05 | 0.400 | 0.039 |
| 17-E04 | 0.241 | 0.016 |
| 17-E05 | 0.220 | 0.015 |
| 32-E04 | 0.623 | 0.046 |
| 32-E05 | 0.641 | 0.028 |
| 85-E04 | 0.151 | 0.008 |
| 85-E05 | 0.134 | 0.009 |
| PC-ESC | 0.390 | 0.012 |

| | | |
|---|---|---|
| Note: the numbers or characters to the left of "-" in Table 7 were the name codes for the dsRNA molecules. | | |

**Table 8. IC₅₀ data of modified dsRNAs in HepG2 cells**

| Name | IC₅₀(nM) |
|---|---|
| 5-E05 | 0.0147 |
| 17-E05 | 0.2273 |
| 85-E04 | 0.0152 |
| 85-E05 | 0.0138 |
| PC-ESC | 0.1497 |

| | |
|---|---|
| Note: the numbers or characters to the left of "-" in Table 8 were the name codes for the dsRNA molecules. | |

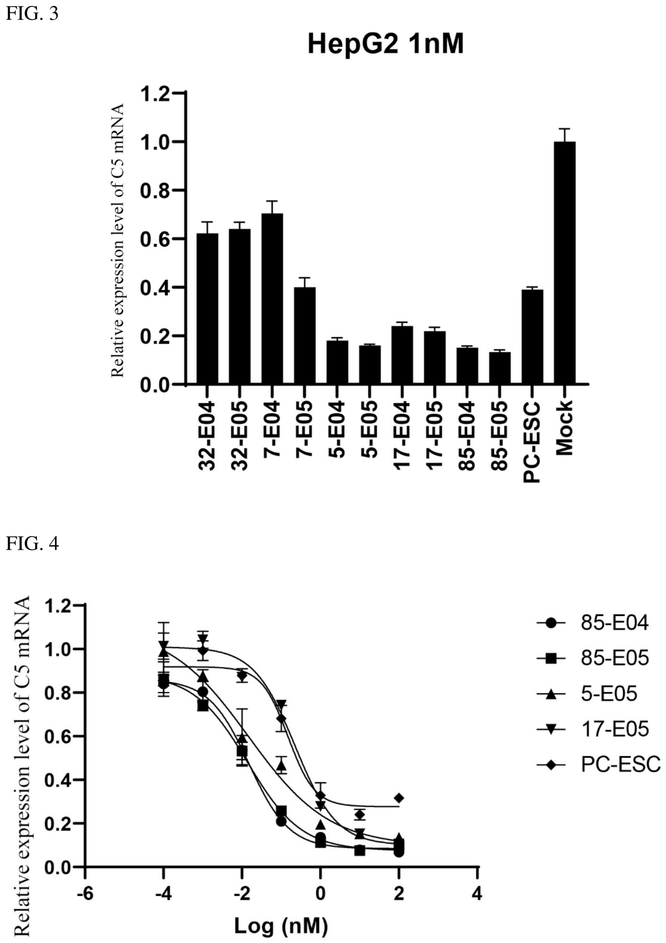

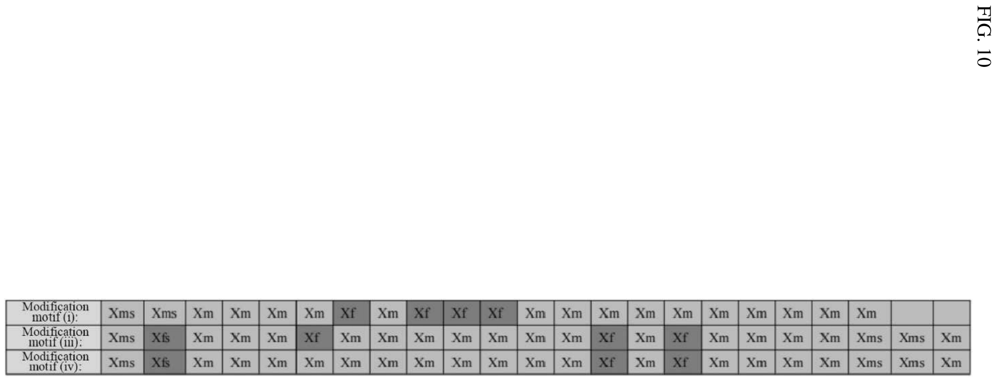

As shown in FIGs. 3 and 4, chemically modified 5-E05, 17-E05, E85-E05 and 85-E04 exhibited higher inhibitory activities in HepG2 cells, which were superior to or equivalent to the positive reference PC-ESC. It demonstrated that the modifications were significant, and some modifications were found to reduce inhibitory activities. In the case of identical modifications in the sense strands and two Modification Motifs (Modification Motifs iii and iv) in the antisense strands, 5-E05, 17-E05 and E85-E05 were superior to 5-E04, 17-E04 and E85-E04, respectively. This demonstrated that maximal 2'-methoxy modification substitutions (Modification Motif iv, see FIG. 10) in the antisense strands effectively improved inhibitory activities.

### Example 3. In vitro Efficacy Tests of GalNAc-dsRNA

The modified dsRNA candidates were conjugated to GalNAc to form GAL-dsRNA complexes. The structures of individual complexes were listed in Table 9. The activities of the complexes were further assessed in primary hepatocytes derived from cynomolgus monkey and HepG2 cells.

### 1. Determination of mRNA expression level in primary hepatocytes derived from cynomolgus monkey

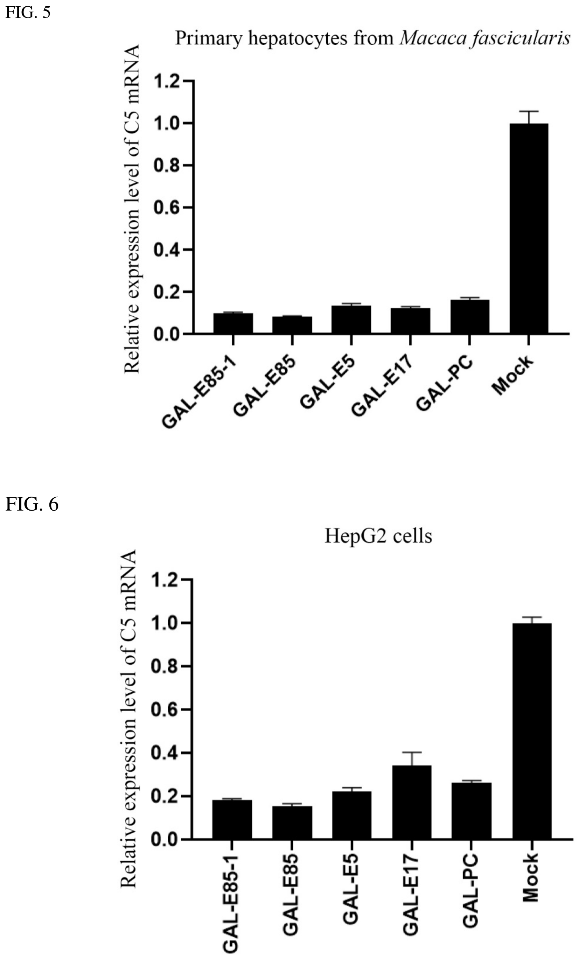

A 24-well plate was pre-coated with collagen I (rat tail), and primary hepatocytes derived from cynomolgus monkey (purchased from Liver Biotechnology) were seeded in the plate at 2×10⁵ cells/well. On the next day, synthesis, transfection, and quantitative PCR assay were performed, using the same procedures as described in Example 1. The final concentration of GalNAc-dsRNA used for transfection was 10 nM, and primer sequences for cynomolgus monkey were listed in Table 3. It can be seen from the results (FIG. 5) that the candidates effectively silenced C5 gene expression in primary hepatocytes derived from cynomolgus monkey.

### 2. Determination of mRNA expression level in HepG2 cells

The procedures were the same as described in Example 1, using GAPDH gene of cynomolgus monkey as the internal reference gene. The primers used for the assays were listed in Table 4. The average target gene expression levels, normalized to the Mock group (set as 1), were shown in Tables 10 and 11.

**Table 9. GalNAc-conjugated dsRNA sequences**

| Name | Naked sequence of sense strand (5' to 3') | Modification motif | Naked sequence of antisense strand (5' to 3') | Modification Motif |
|---|---|---|---|---|
| GAL-E5 | SEQ ID NO: 5 | Motif i | SEQ ID NO: 105 | Motif iv |
| GAL-E17 | SEQ ID NO: 17 | Motif i | SEQ ID NO: 117 | Motif iv |
| GAL-E85-1 | SEQ ID NO: 85 | Motif i | SEQ ID NO: 185 | Motif iii |
| GAL-E85 | SEQ ID NO: 85 | Motif i | SEQ ID NO: 185 | Motif iv |
| GAL-PC | SEQ ID NO: 99 | Motif ii | SEQ ID NO: 100 | Motif v |

| | | | | |
|---|---|---|---|---|
| Note 1: The number to the right of "E" in Table 9 was the name code, and "PC" in "GAL-PC" was the name code. Note 2: The 3' ends of the sense strands of all sequences in Table 9 were conjugated to L96 ligand. | | | | |

**Table 10. Single-dose screening results of GalNAc-conjugated dsRNAs in primary hepatocytes derived from cynomolgus monkey**

| Name | C5 mRNA relative expression level (10 nM) | standard error (±SEM) |
|---|---|---|
| GAL-E5 | 0.136 | 0.011 |
| GAL-E17 | 0.124 | 0.008 |
| GAL-E85-1 | 0.101 | 0.004 |
| GAL-E85 | 0.084 | 0.003 |
| GAL-PC | 0.163 | 0.011 |

| | | |
|---|---|---|
| Note: The number to the right of "E" in Table 10 was the name code, and "PC" in "GAL-PC" was the name code. | | |

**Table 11. Single-dose screening results of GalNAc-conjugated dsRNAs in HepG2 cells**

| Name | C5 mRNA relative expression level (1 nM) | standard error (±SEM) |
|---|---|---|
| GAL-E5 | 0.222 | 0.017 |
| GAL-E17 | 0.344 | 0.060 |
| GAL-E85 | 0.154 | 0.011 |
| GAL-E85-1 | 0.180 | 0.008 |
| GAL-PC | 0.260 | 0.013 |

| | | |
|---|---|---|
| Note: The number to the right of "E" in Table 11 was the name code, and "PC" in "GAL-PC" was the name code. | | |

As shown in FIGs. 5 and 6, the candidate GAL-E5, Gal-E85 and Gal-E85-1 effectively silenced C5 expression in both cells, demonstrating superior activity compared to the positive reference GAL-PC.

### Example 4. In vivo Efficacy Tests

6 to 8-week-old SPF-grade humanized C5 mice (available from Shanghai Model Organisms Center, Inc.) were randomly divided into groups (n=10 per group, 1:1 male:female ratio), and were administered with a single subcutaneous injection (dosage regimens shown in Tables 13 and 14). Serum samples were collected via retro-orbital bleeding at Day -3 (before administration), 3, 7, 12, 19, 26, 33, 40, and 54 (the first administration was on Day 0) for determination of C5 protein content.

**Table 12. 1 mpk single-dose regimen**

| Name | Administration dosage | humanized C5 mice | Bleeding time |
|---|---|---|---|
| Blank | normal saline | N=10, five males and five females | Day -3, 3, 7, 12, 19, 26, 33, 40, 54 |
| GAL-E5 | 1mpk | | |
| GAL-E17 | 1mpk | | |
| GAL-E85-1 | 1mpk | | |
| GAL-E85 | 1mpk | | |
| GAL-PC | 1mpk | | |

| | | | |
|---|---|---|---|
| Note: The number to the right of "E" in Table 12 was the name code, and "PC" in "GAL-PC" was the name code. | | | |

**Table 13. Dose gradient single-dose regimen**

| Name | Administration dosage | C5-humanized mice | Bleeding time |
|---|---|---|---|
| Blank | normal saline | N=10, five males and five females | Day -3, 3, 12, 19, 26, 33, 40, 54, 61, 68, 75, 82, 89, 96, 103 |
| GAL-PC | 0.3mpk | | |
| | 1mpk | | |
| | 3mpk | | |
| GAL-E5 | 0.3mpk | | |
| | 1mpk | | |
| | 3mpk | | |
| GAL-E85 | 0.3mpk | | |
| | 1mpk | | |
| | 3mpk | | |

| | | | |
|---|---|---|---|
| Note: The number to the right of "E" in Table 13 was the name code and "PC" in "GAL-PC" was the name code. | | | |

### ELISA assay of C5 protein

### 1. Method

The serum samples collected from each group were diluted at 1:20,000 and residual C5 contents were measured using an ELISA kit (ab125963) according to the Manufacturer's instructions. Absorbance was measured at 450 nm using a microplate reader. The relative residual C5 contents in individual groups were calculated in relative to that of the blank control group and statistical analysis was done using a GraphPad software.

### 2. Results

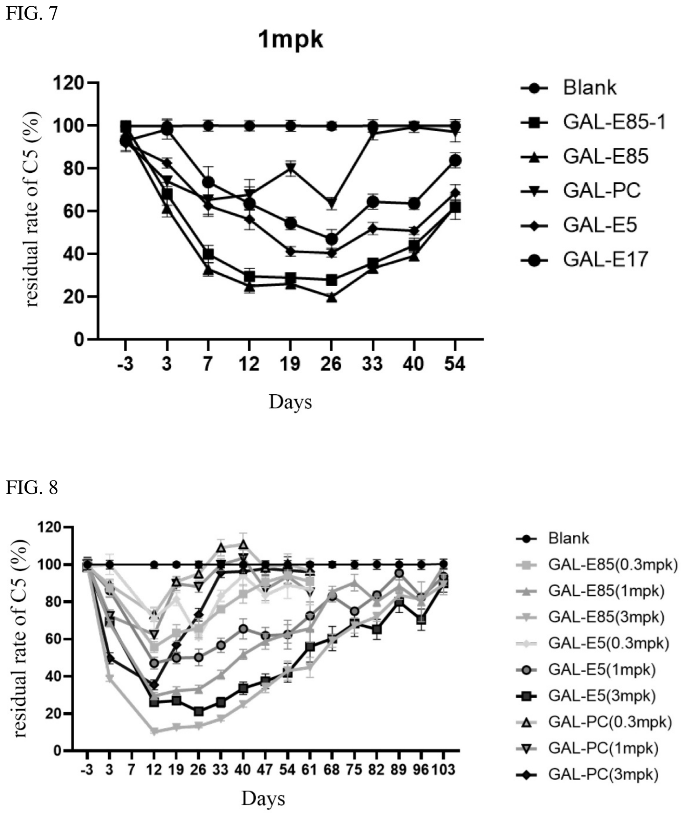

The results (FIG. 7) demonstrated that after a single 1 mpk dose, serum C5 contents from the mice administrated with the four dsRNA molecule candidates were significantly lower than that from the Blank control group, and the candidates exhibited superior efficacy compared to the positive reference. It was demonstrated in the further dose-response studies (FIG. 8) that GAL-E85 (0.3 mpk) returned to the pre-dose point by Day 47, and GAL-E85 (1 mpk or 3 mpk) sustained for a longer time, up to Day 103; while the positive reference GAL-PC (3 mpk) returned to the baseline by Day 33. Meanwhile, GAL-E5 exhibited significantly greater potency than GAL-PC. In summary, the dsRNA molecule candidates were demonstrated to exhibit superior inhibitory efficacy against C5 compared to the positive reference, i.e., Cemdisiran (AD-62643) currently at the clinical stage, indicating therapeutic potential for treating C5-related diseases.

### Example 5: In Vivo Efficacy Tests in cynomolgus monkey

Adult male cynomolgus monkeys (n= 3 per group) were administered via a single subcutaneous injection in this study (dose groups shown in Table 14). Day -3 was designated as the pre-dose time point and Day 0 as the dosing time point. Blood samples were collected at the designated time points. C5 protein contents in collected sera were quantified using an ELISA kit (ab125963) as described in Example 4. Residual rate of C5 in each group was statistically analyzed relative to the pre-dose point.

**Table 14. 3 mpk single dose regimen**

| Name | Administration dose | cynomolgus monkey | Blood sampling time |
|---|---|---|---|
| GAL-E5 | 3 mpk | N=3, male | Day -3, 3, 7, 14, 21, 28, 32, 35, 39, 42, 45, 49, 53, 56 |
| GAL-E85 | 3 mpk | | |
| GAL-PC | 3 mpk | | |

| | | | |
|---|---|---|---|
| Note: The number to the right of "E" in Table 14 was the name code, and "PC" in "GAL-PC" was the name code. | | | |

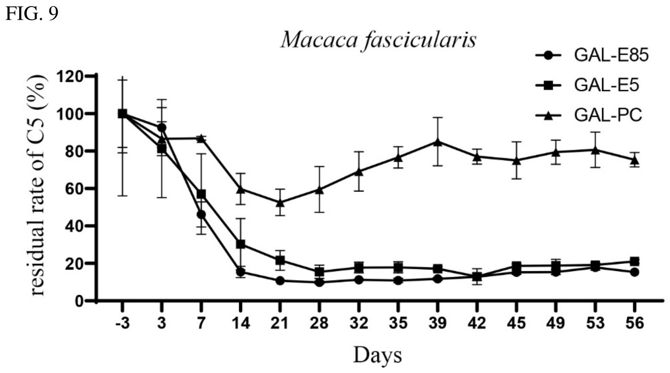

The results (FIG. 9) demonstrated that compared to the positive reference GAL-PC, the candidates GAL-E85 and GAL-E5 exhibited stronger and more sustained C5 protein inhibition. The maximal inhibition rate achieved by GAL-PC was 47.5%, while GAL-E85 and GAL-E5 reached 90.2% and 85.5%, respectively.

In summary, it can be seen that, when the sense strand of the dsRNAs of the present invention comprises the modifications as defined in Motif i, dsRNAs comprising the antisense strand comprising the modifications as defined in Motif iv lead to superior *in vivo* and *in vitro* inhibitory efficacy as compared with dsRNAs comprising the antisense strand comprising the modifications as defined in Motif iii.

The foregoing description merely illustrates preferred embodiments of the present invention, which serve as examples and do not limit the essential feature combinations required to practice the inventions in the present application. The section headings provided are not intended to limit the various embodiments of the present application. Terms such as "comprising", "containing", and "including" are not intended to limit. Unless otherwise specified, unmodified nouns encompass plural forms, and the terms "or" and "either" shall be understood to mean "and/or". Unless expressly defined herein, all technical and scientific terms used herein shall have the meanings commonly understood by a person skilled in the art. All publications and patents cited herein are incorporated by reference. Without departing from the spirit and scope of the present application, various modifications and variations of the described methods and compositions will be apparent to those skilled in the art. Although the present application has been described with reference to specific preferred embodiments, it should be understood that claimed inventions should not be unduly limited to these particular implementations. Indeed, various variations for carrying out the described modes of the present application that would be apparent to a person skilled in the art are intended to be included in the scope of the appended claims.

## Claims

1. A dsRNA molecule for inhibiting the gene expression of C5, comprising a sense strand and an antisense strand that are complementary to form a double-stranded region, wherein the sense strand and/or the antisense strand comprises or consists of 15 to 25 nucleotides, the antisense strand is complementary to at least 15, 16, 17, 18, 19, 20, or 21 contiguous nucleotides in the sequence as set forth in any one of SEQ ID NOs: 1 to 98, the double-stranded region has a length of 15 to 25 bp, and at least one nucleotide in the dsRNA molecule comprises a modification.

2. The dsRNA molecule according to claim 1, wherein the nucleotide sequence of the sense strand is as set forth in any one of SEQ ID NOs: 1 to 98, and the sense strand nucleotide sequences as set forth in SEQ ID NOs: 1-98 of the dsRNA molecules have corresponding antisense strand nucleotide sequences in SEQ ID NOs: 101 to 198 in sequence.

3. The dsRNA molecule according to claim 1 or 2, wherein the modification is one or more selected from the group consisting of a locked nucleic acid modification, an open or unlocked nucleic acid modification, a 2'-methoxyethyl modification, a 2'-O-methyl modification, a 2'-O-allyl modification, a 2'-C-allyl modification, a 2'-fluoro modification, a 2'-deoxy modification, a 2'-hydroxyl modification, a phosphorothioate backbone modification, a DNA modification, a fluorescent probe modification, and a ligand modification.

4. The dsRNA molecule according to claim 3, wherein the dsRNA molecule comprises modifications, wherein:
(i) the sense strand has a length of 17 to 21 nt, preferably 21 nt, the sense strand consists of alternating 2'-O-methyl modified regions and 2'-fluoro modified regions, wherein each modified region is 1 to 3 nucleotides in length, and the sense strand has identical modifications for the first modified region from the 5' end and the first modified region from the 3' end; and
(ii) the antisense strand has a length of 19 to 23 nt, preferably 23 nt, the antisense strand consists of alternating 2'-O-methyl modified regions, 2'-fluoro modified regions, unmodified regions or DNA regions, wherein each modified region is 1 to 5 nucleotides in length; and the antisense strand has phosphorothioate backbone linkages among the contiguous nucleotides at positions 2 to 5 from the 5' end and among the contiguous nucleotides at positions 1 to 3 from the 3' end; and
preferably,
(i) the sense strand has a length of 21 nt, has 2'-fluoro modifications at positions 7 and 9 to 11 from the 5' end and 2'-O-methyl modifications at the remaining positions, and has phosphorothioate backbone linkages among the contiguous nucleotides at positions 1 to 3 from the 5' end; and
(ii) the antisense strand has a length of 23 nt, has 2'-fluoro modifications at positions 2, 14, and 16 and optional position 6 from the 5' end and 2'-O-methyl modifications at the remaining positions, and has phosphorothioate backbone linkages among the contiguous nucleotides at positions 1 to 3 from the 5' end and among the contiguous nucleotides at positions 1 to 3 from the 3' end.

5. The dsRNA molecule according to any one of claims 1 to 4, wherein the naked sequence of the sense strand of the dsRNA molecule is as set forth in (A1), (A2) or (A3):
(A1): GUGAUUCAAGUUUAUGGAUAC (SEQ ID NO: 5),
(A2): UGUGUAUUUGGAAGUUGUAUC (SEQ ID NO: 17), or
(A3): GUGAAGAAAUGUUGUUACGAU (SEQ ID NO: 85); and
the naked sequence of the antisense strand of the dsRNA molecule is as set forth in(A4), (A5) or (A6):
(A4): GUAUCCAUAAACUUGAAUCACAA (SEQ ID NO: 105),
(A5): GAUACAACUUCCAAAUACACAUA (SEQ ID NO: 117), or
(A6): AUCGUAACAACAUUUCUUCACUA (SEQ ID NO: 185).

6. The dsRNA molecule according to claim 5, wherein:
the sense strand of the dsRNA molecule comprises the modifications as defined in Modification Motif i:
Modification Motif i:
XmsXmsXmXmXmXmXfXmXfXfXfXmXmXmXmXmXmXmXmXmXm; and
the antisense strand of the dsRNA molecule comprises the modifications as defined in Modification Motif iii or Modification Motif iv:
Modification Motif iii:
XmsXfsXmXmXmXfXmXmXmXmXmXmXmXfXmXfXmXmXmXmXmsXmsXm, or
Modification Motif iv:
XmsXfsXmXmXmXmXmXmXmXmXmXmXmXfXmXfXmXmXmXmXmsXmsXm;
wherein, for each Modification Motif, individual "X's" represent ribonucleotides from the 5' end to the 3' end of the sense strand in sequence, "Xm" represents a ribonucleotide comprising a 2'-O-methyl modification, "Xf" represents a ribonucleotide comprising a 2'-fluoro modification, and "s" represents a phosphorothioate backbone linkage between two flanking ribonucleotides.

7. The dsRNA molecule according to claim 6, wherein the dsRNA molecule further comprises a ligand conjugated to the 3' end of the sense strand, and the ligand is L96 having the structure of Formula I:

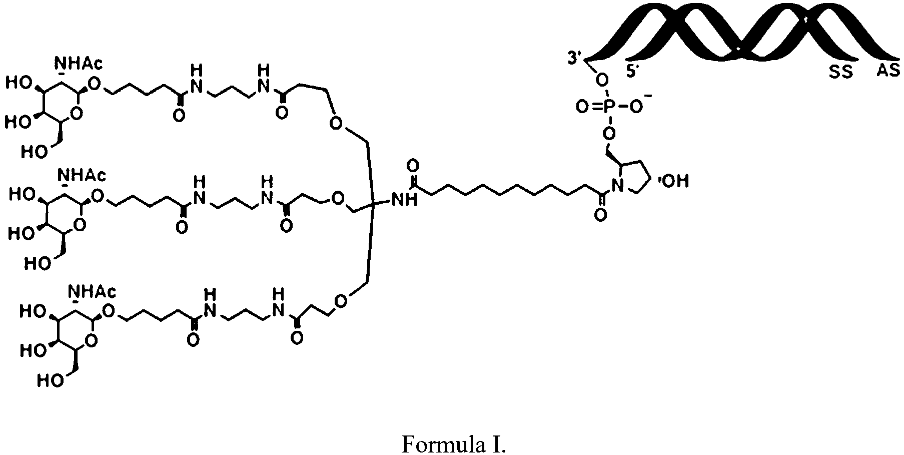

8. The dsRNA molecule according to any one of claims 5 to 7, wherein the sense strand comprises the modifications as defined in Modification Motif i, and the antisense strand comprises the modifications as defined in Modification Motif iv.

9. A pharmaceutical composition comprising the dsRNA molecule according to any one of claims 1 to 8, and a pharmaceutically acceptable excipient; preferably, the pharmaceutical composition further comprises an additional compound that inhibits complement proteins or complement protein expressions.

10. Use of the dsRNA according to any one of claims 1 to 8 or the pharmaceutical composition according to claim 9 for any one selected from the group consisting of:
(i) inhibiting the gene expression of complement C5 or manufacturing a product for inhibiting the gene expression of complement C5;
(ii) manufacturing a product for reducing C5 protein in a serum;
(iii) preventing and/or treating a disease mediated by complement C5 gene or manufacturing a product for preventing and/or treating a disease mediated by complement C5 gene; and
(iv) alleviating symptoms of a disease mediated by complement C5 gene or manufacturing a product for alleviating symptoms of a disease mediated by complement C5 gene;
wherein the disease mediated by complement C5 gene is one or more selected from the group consisting of ophthalmic diseases, including dry/wet age-related macular degeneration (AMD), and geographic atrophy (GA); hematologic diseases, including paroxysmal nocturnal hemoglobinuria (PNH), and thrombotic microangiopathies (TMAs); cardiovascular diseases; autoimmune diseases, including rheumatoid arthritis, and lupus erythematosus; renal diseases, including typical hemolytic uremic syndrome (aHUS), C3 glomerulopathy (C3G), IgA nephropathy and lupus nephritis; neurological diseases, including Alzheimer's disease (AD), and myasthenia gravis (gMG); neoplastic diseases, including complement-associated liver cancer or lung cancer; surgery-related diseases, including hepatic ischemia-reperfusion injury, burn rehabilitation, and post-transplant rejection; and respiratory diseases, including asthma, idiopathic pulmonary fibrosis, and chronic obstructive pulmonary disease (COPD).

11. A double-stranded nucleic acid for inhibiting the gene expression of C5, or a pharmaceutically acceptable salt thereof, wherein the double-stranded nucleic acid comprises a first strand and a second strand, wherein:
the first strand is AUCGUAACAACAUUUCUUCACUA (SEQ ID NO: 185), and the second strand is GUGAAGAAAUGUUGUUACGAU (SEQ ID NO: 85); or
the first strand is GUAUCCAUAAACUUGAAUCACAA (SEQ ID NO: 105), and the second strand is GUGAUUCAAGUUUAUGGAUAC (SEQ ID NO: 5); and
the first strand has the modifications as defined in Modification Motif iv:
Modification Motif iv:
XmsXfsXmXmXmXmXmXmXmXmXmXmXmXfXmXfXmXmXmXmXmsXmsXm; and
the second strand is conjugated to an L96 ligand at the 3' end and has the modifications as defined in Modification Motif i:
Modification Motif i:
XmsXmsXmXmXmXmXfXmXfXfXfXmXmXmXmXmXmXmXmXmXm;
wherein "Xm" represents a 2'-O-methyl modified ribonucleotide A, U, C or G, "Xf" represents a 2'-fluoro modified ribonucleotide A, U, C or G, "s" represents a phosphorothioate backbone linkage between two flanking ribonucleotides, and the 3' end conjugation of the second strand to the L96 ligand is shown below:

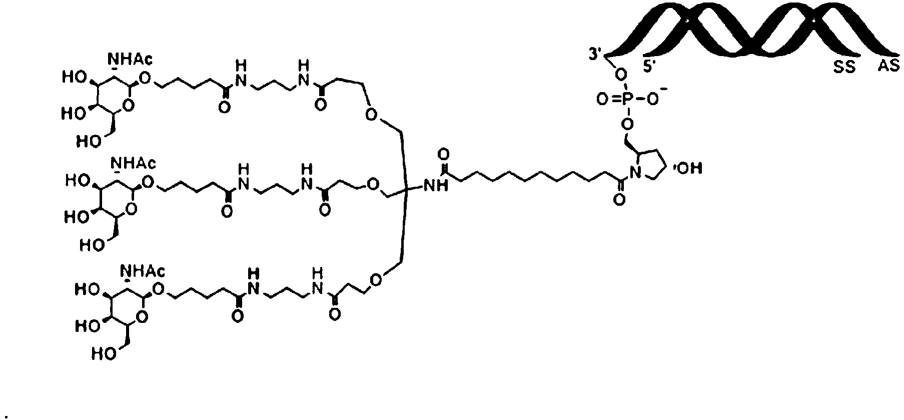

12. A pharmaceutical composition comprising the double-stranded nucleic acid according to claim 11, and a pharmaceutically acceptable carrier.
